Europäisches Patentamt

European Patent Office

Office européen des brevets

(19)

(11) Publication number: **0 109 657**
**A1**

# EUROPEAN PATENT APPLICATION

(21) Application number: **83111462.4**

(22) Date of filing: **17.11.83**

(51) Int. Cl.³: **A 61 M 25/00**
**B 29 F 1/10**

(30) Priority: **18.11.82 US 442694**

(43) Date of publication of application:
**30.05.84 Bulletin 84/22**

(84) Designated Contracting States:
**AT BE CH DE FR GB IT LI LU NL SE**

(71) Applicant: **ABBOTT LABORATORIES**
**14th Street and Sheridan Road**
**North Chicago, Illinois 60064(US)**

(72) Inventor: **Zaha, Jürgen Heinz**
**Rt. 1, P. O. Box 453**
**Moore South Carolina 29369(US)**

(72) Inventor: **Best, Robert Joseph**
**144 Timberlake Drive**
**Inman South Carolina 29349(US)**

(72) Inventor: **Lundberg, Kenneth Leslie**
**174 Henson Street**
**Spartanburg South Carolina 29302(US)**

(74) Representative: **Josif, Albert et al,**
**MODIANO, JOSIF, PISANTY & STAUB Via Meravigli, 16**
**I-20123 Milan(IT)**

(54) **Improved catheter assembly.**

(57) An improved catheter assembly (10) comprises an insert molded catheter hub (12) and catheter (14). The catheter has an axial bore (18) and a flared tip portion (16) about its proximal end. The catheter hub has a lumen (20) extending therethrough with the flexible catheter fixedly positioned within the lumen. In order to accomplish this, the catheter hub has a chamber (22) integrally formed therein about the flared tip of the catheter, which tightly grips and seals the catheter but allows the flow of fluid through the lumen of the catheter hub and the axial bore of the catheter.

The invention further comprises a method of manufacturing the catheter. The method comprises the steps of cutting a section of catheter tubing, flaring one end against a heated die. The catheter is then mounted on a core pin, and inserted into an injection mold. The mold is closed and heated plastic material is injected in the cavity and about the flared end of the catheter. After cooling, the catheter assembly is removed from the mold, and then from the core pin.

Fig. 1.

EP 0 109 657 A1

IMPROVED CATHETER ASSEMBLY

Background of the Invention

This invention relates generally to catheter assemblies and more particularly to a unique hub and catheter securement structure that greatly facilitates the fabrication of the catheter assembly and improves the adherence of the catheter to the catheter hub when in use.

Catheter assemblies utilizing a relatively long, flexible, hollow plastic catheter for insertion into the vein of a patient have long been utilized for infusion of intravenous fluids and other medication. A catheter assembly of the type contemplated by the subject invention is illustrated and described in U.S. Patent No. 3,094,122. Another infusion catheter assembly is illustrated and described in U.S. Patent No. 3,802,433. This latter patent discloses that a flexible, plastic catheter may be secured to the internal bore of a catheter hub by inserting a metal sleeve internally of the proximal end of the catheter to expand that end against the internal surface of the catheter hub. A third relevant example of prior art is European patent application No. 0001106 which describes a catheter hub having an axial opening therethrough and a catheter securement insert which is inserted into the end of the catheter to hold it within the hub. Many such approaches for the attachment of a plastic catheter to a plastic or metal hub have been previously suggested. However, none of the prior art devices have solved the essential problem of attaching the catheter directly to the catheter hub without the use of a separate insert. This is desirable both from the standpoint of reducing cost and in improving the quality of the product.

## Summary of the Invention

The present invention relates to a catheter assembly having an improved, insert-molded catheter hub and catheter. The catheter has a flared proximal end and an axial bore extending therethrough. The catheter hub has a lumen extending therethrough with a flexible catheter fixedly positioned within the lumen. This is accomplished in part by a chamber integrally formed in the interior of the catheter hub about the flared proximal end of the catheter, which tightly grips and seals the catheter about the periphery of the flared proximal end, but allows the flow of fluid through the lumen of the catheter hub and the axial bore of the catheter.

The chamber described above is preferably contained within an annular projection, integrally formed and circumferentially disposed about the catheter hub. As a result of this annular projection or bulge in the wall of the catheter hub, the flared proximal end of the catheter within the chamber may be encapsulated on three sides.

While the periphery of the flared proximal end of the catheter is encapsulated by the walls of the chamber, the bore of the catheter is unobstructed and open to the lumen of the catheter hub. As a result, fluid is allowed to flow through the lumen during use, but seepage of fluid around the flared proximal end of the catheter is prevented.

An additional feature of the invention is a pinched waist portion integrally formed in the catheter hub immediately behind the chamber. The pinched waist portion causes the rear portion of the chamber to be reduced in inside diameter, thereby abutting against the flared proximal end of the catheter so as to prevent backward movement of the catheter during venipuncture.

An additional feature of the invention is a substantially tubular projection integrally formed and extending from the distal end of the catheter hub. The tubular projection has a reduced wall thickness relative to the remainder of the catheter hub. This reduced wall thickness reduces the amount of shrinkage which occurs during manufacture. As a result, by controlling the size of the tubular projection, the grip of the tubular projection around the catheter is maintained after manufacture.

In a preferred embodiment, the lumen of the catheter hub decreases in inside diameter proximate the flared proximal end of the catheter so that the area behind and around the flared proximal end is filled with the material of the wall of the chamber, thereby encapsulating the proximal end. The lumen of the catheter hub may also conically taper proximate the proximal end of the catheter hub. This taper facilitates the telescopic insertion of and connection to a needle hub having a needle affixed therein. By controlling the shape of the needle hub and the shape of the lumen, the needle may be positioned through the catheter hub and catheter with the point of the needle extending sufficiently from the catheter to align with the bevel of the catheter tip, thereby forming a smooth transition between the tip of the catheter and the point of the needle.

An additional feature of the invention is a plurality of radial serrations axially disposed about the periphery of the catheter hub. These serrations are designed to facilitate gripping of the catheter hub during use. The serrations also vent heat from the molded plastic hub after manufacture, thereby reducing internal shrinkage of the hub after manufacture.

The invention encompasses catheters of from 14 to 26 gauge in thickness. When the catheter is from 18 to 26 gauge in thickness, the catheter hub in the area around the chamber ranges from .025 − .035" plus or minus .005 depending on gauge thickness. This wall configuration has been shown to reduce shinkage of the catheter hub away from the catheter hub following molding. When the catheter is from 14 or 16 gauge in thickness, the catheter hub is .036 plus or minus .005 inches in wall thickness proximate the chamber. Again, this dimension reduces shrinkage of the catheter hub away from the catheter following molding. The catheter hub is preferably injection molded of a medical grade plastic material, such as acrylonitrile-butadiene-styrene (ABS). The catheter is preferably constructed of a medical grade tube of polyethylene terapthalate (PET).

The invention further comprises a method of manufacturing the previously mentioned catheter hub and catheter. The method comprises the steps of cutting a section of catheter tubing to a desired length and abutting one end of the tubing against a heated die. The heated die has a flat base with a conical appendage extending therefrom. This causes the end of the catheter to flare outward and curl over. When the catheter is sufficiently flared, it is removed from contact with the heated die. The catheter is then mounted, flared end first, on a tapered core pin. The core pin has an upper section of substantially the same outside diameter as the inside diameter of the catheter. The lowered tapered section is of increasing outside diameter so as to prevent further downward movement of the catheter. The catheter and core pin are then inserted into an injection mold having a cavity shaped as a catheter hub with an enlarged chamber formed in the cavity. When properly positioned, the flared end of the catheter is positioned in the chamber. The mold is then closed and a quantity of heated plastic material is injected into the cavity and about the flared first end of the catheter and the adjacent proximal portion of the catheter. The plastic material is then cooled so as to form a catheter hub about the catheter which mechanically grips the catheter. The mold is then opened and the core pin, catheter hub and catheter are removed. The catheter hub and catheter are then removed from the core pin.

An additional feature of the invention is positioning the gate of the injection mold at a 45° angle to the chamber. This forces the catheter upward on the core pin and into its proper position against the tapered section of the core pin, when heated plastic material is injected. As a result, the flared end of the catheter is encapsulated in heated plastic material. The remainder of the cavity is then filled with heated plastic material to form the catheter hub. This filling of the chamber before filling the remainder of the cavity is accomplished by decreasing the inside diameter of the cavity proximate and immediately behind the chamber. As a result, as plastic flows into the chamber, its flow beyond the chamber is constricted. This causes the heated plastic to accumulate and be compressed about the flared end of the catheter which causes the resulting catheter hub to tightly grip the flared end. Once the chamber has been filled, additional heated plastic injected into the cavity is forced into the remainder of the cavity, thereby completing formation of the catheter hub.

An additional feature of the invention is flaring of the catheter in a hexagonal configuration. This is accomplished by constructing the conical appendage in the previously mentioned heated die in a hexagonal shape. The flared periphery of the catheter is formed in a hexagonal configuration by telescopic insertion of the first end of the catheter tubing over the hexagonal, conical appendage. The hexagonal configuration of the resulting flared catheter facilitates gripping of the flared end of the catheter by the catheter hub. In certain preferred embodiments, the flared end may also be formed in a C-shaped configuration in cross section. This C-shape facilitates encapsulation on the forward surface of the flared end, thereby preventing axial movement of the catheter during use. An additional means of improving encapsulation is the thickening of the wall portion proximate the flared proximal end of the catheter. This increases the compression of the catheter against the interior of the chamber, thereby more tightly gripping the catheter.

Brief Description of the Drawings

FIGURE 1 of the drawings is a vertical section of the improved catheter and catheter hub of the present invention.

FIGURE 2 of the drawings is a side view of the improved catheter and catheter hub of FIGURE 1.

FIGURE 3 of the drawings is a vertical section of a flaring tool used to flare the proximal end of the improved catheter of the present invention.

FIGURE 3A is an end view of a catheter having a hexagonally flared proximal end.

FIGURE 3B is a vertical section of the proximal end of a catheter flared in a C-shaped sectional configuration.

FIGURE 3C is a perspective view of an alternative embodiment of a flaring tool with the catheter tubing shown in section.

FIGURE 4 of the drawings is a top view of a hexagonal flaring tool.

FIGURE 4A is a side view of the hexagonal flaring tool of FIGURE 4 with a catheter inserted therein.

FIGURE 4B is a vertical section of the hexagonal flaring tool and catheter of FIGURE 4A.

FIGURE 5 of the drawings is a side view of the catheter of FIGURE 1 affixed on a core pin.

FIGURE 6 of the drawings is a side view of the core pin inserted into an injection mold prior to closing of the mold.

FIGURE 7 of the drawings is a vertical section of the mold after closing and after injection of heated plastic material.

FIGURE 8 of the drawings is a schematic diagram showing from left to right: cutting of a catheter, placement of the catheter on a core pin, insertion of the catheter and core pin into a mole, injection of heated plastic material into the mold which causes the catheter to be moved slightly backward on the core pin, followed by encapsulation of the flared end of the catheter by the heated plastic material, and removal of the catheter and hub from the mold.

Detailed Description of the Preferred Embodiment

While this invention is susceptible of embodiment in many different forms, there is shown in the drawings and will herein be described in detail, several specific embodiments, with the understanding that the present disclosure is to be considered as an exemplification of the principles of the invention and is not intended to limit the invention to the embodiments illustrated.

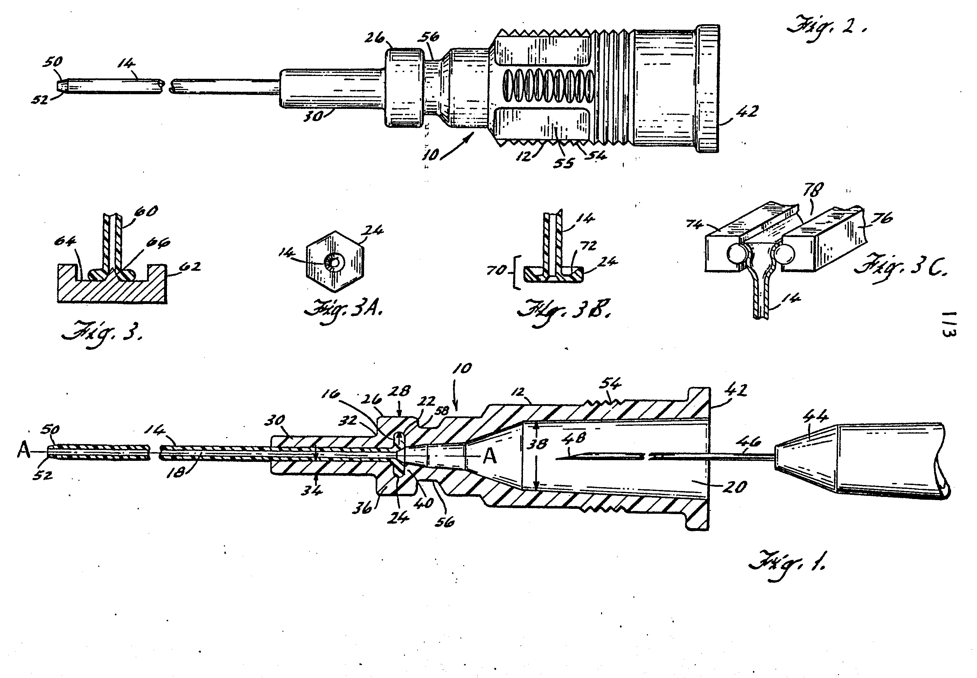

As best seen in FIGURE 1 of the drawings, an improved catheter assembly generally 10 comprises an insert molded catheter hub 12 having a catheter 14 contained therein. Flexible catheter 14 has a flared proximal end 16 and an axial bore 18 extending therethrough along axis AA. Catheter hub 12 also has a lumen 20 extending axially therethrough along axis AA. Flexible catheter 14 is fixedly positioned within lumen 20 by means of a chamber 22 integrally formed in lumen 20 about flared proximal end 16. Chamber 22 tightly grips and seals catheter 14 about the exterior 24 of flared proximal end 16, but allows the flow of fluid through lumen 20 and through axial bore 18.

An integral part of insert molded catheter hub 12 is annular projection 26 integrally formed and circumferentially disposed about catheter hub 12. Annular projection 26 contains chamber 22 therein. By means of annular projection 26, the wall thickness 28 of catheter hub 12 about flared proximal end 16 and thereby the encapsulation of flared proximal end 16 within chamber 22 may be precisely controlled. By this it is meant that by using a desired wall thickness 28, the amount of shrinkage of the catheter hub 12 after molding is precisely controlled, thereby maintaining a tight fit between the interior of chamber 22 and flared proximal end 16.

An additional means of affixing catheter hub 12 to catheter 14 is tubular projection 30 which is integrally formed and extends from distal end 32 of catheter hub 12. Tubular projection 30 is of a reduced wall thickness 34 relative to the remainder of catheter hub 12 so as to shrink less after manufacture than the remainder of catheter hub 12, thereby improving gripping of catheter 14 by catheter hub 12.

In a preferred embodiment, the exterior 24 of flared proximal end 16 is encapsulated by the walls 36 of chamber 22. However, the axial bore 18 of catheter 14 is unobstructed and open to lumen 20 of catheter hub 12. As a result, fluid is allowed to flow through lumen 20. At the same time, passage of fluid about the exterior 24 of flared proximal end 16 is prevented.

An additional feature of catheter hub 12 is the structure of inside diameter 38 of lumen 20. Inside diamater 38 decreases in diameter proximate flared proximal end 16 so that the area 40 behind and around flared proximal end 16 is filled with the material of the wall 36 of chamber 22. As a result, flared proximal end 16 is encapsulated. An additional design feature of lumen 20 is proximal end 42 of catheter hub 12. Lumen 20 conically tapers proximate proximal end 42 of catheter hub 12. This conical taper facilitates telescopic insertion of and connection to a tapered needle hub 44 having a needle 46 affixed therein. Needle 46 is positioned through catheter hub 12 and catheter 14 with the point 48 of needle 46 extending sufficiently from catheter 12 to align with the bevel 50 of the distal end 52 of catheter 14, thereby forming a smooth transition with the distal end 52 of catheter 12 and the point 48 of needle 46.

An additional feature of the invention is a pinched waist portion 56 integrally formed in catheter hub 12 proximate and immediately behind chamber 22. Pinched waist portion 56 causes the rear portion 58 of chamber 22 to be reduced in inside diameter, thereby abutting against flared proximal end 16. This pinched waist portion 56 thereby prevents backward movement of catheter 14 during venipuncture.

An additional feature of the device is a series of radial serrations 54 axially disposed about the periphery of catheter hub 12. These radial serrations are constructed and arranged for gripping of catheter hub 12 during use.

The previously mentioned catheter usually comes in gauges from 14 through 26 gauge (French gauge .081 - .026" OD), usually increasing in increments of 2 (i.e., 14, 16, 18, etc.). When catheter 14 is constructed from 18 to 26 gauge in thickness, catheter hub 12 ranges from .025 - .035" plus or minus .005 depending on gauge thickness of walls 28 of walls 36 of chamber 22. This particular wall thickness reduces shrinkage of catheter hub 12 away from catheter 14 following molding. When catheter 14 is from 14 or 16 gauge in thickness, catheter hub 12 is .036 plus or minus .005 inches in wall thickness 28. As a result of this construction, the wall thickness 28 of catheter hub 12 minimizes shrinkage of the walls 36 of catheter hub 12 away from catheter 14 following molding, thereby improving gripping of the catheter.

In a preferred embodiment, catheter hub 12 is injection molded of a medical grade-thermoplastic material such as acrylinitrile-butadiene-styrene, polypropylene, polyethylene, or other commonly known theremoplastics. Catheter 14 is constructed of conventional catheter material such as polyethylene terraphthalate, silicone elastomer, polyvinyl chloride or other commonly known catheter materials.

In an alternative embodiment of the invention, not shown, catheter hub 12 may be bifurcated and joined about catheter 14 by adhesive or heat sealing means. Similarly, flared proximal end 16 may be heat sealed to chamber 22 in those cases where the catheter material is heat sealable. Again, alternatively, catheter 14 may be adhesively secured to catheter hub 12; however, this is a relatively difficult, expensive and messy procedure.

As best seen in FIGURES 3, 3A, 3B, and 3C of the drawings the present invention also comprises a method of manufacturing a catheter 14 having a flared end 16. A section of catheter tubing 60 is abutting the proximal end 16 against a heated die 62 having a flat bore 64 with a conical appendage 66 extending perpendicularly therefrom. This causes the proximal end 16 of catheter 14 to flare outwardly and curl over, as best seen in FIGURE 3B. Once the flared proximal end 16 is curled, the catheter 14 is removed from contact with heated die 62. As a result, catheter 14 has a proximal end 16 flared about its exterior 24.

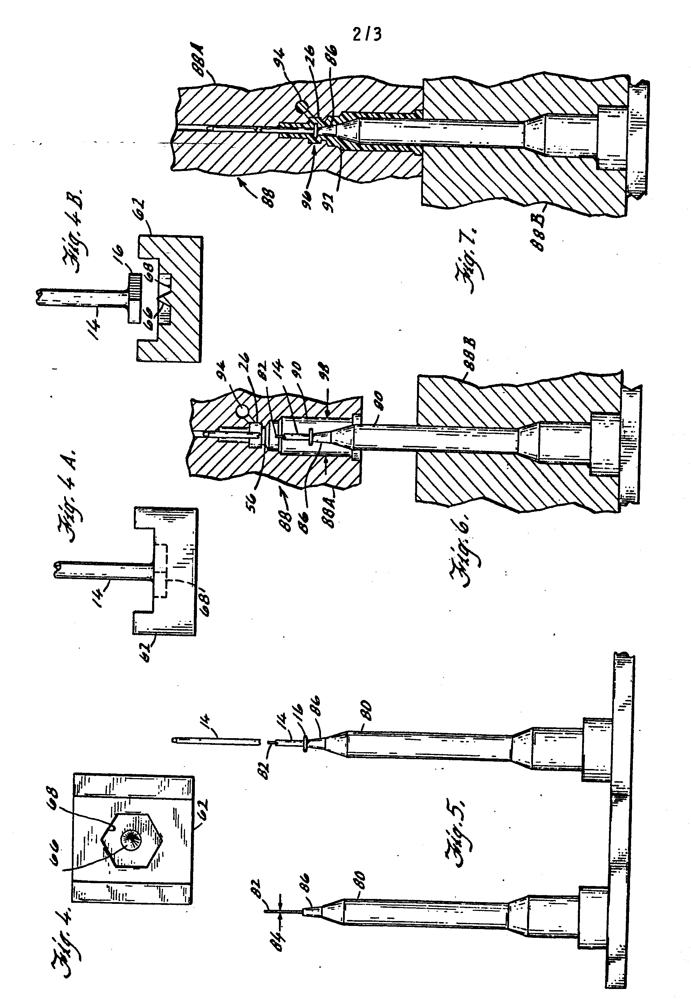

An additional feature of the present invention is the manufacture of flared proximal end 16 in a hexagonal shape. As seen in FIGURES 3A, 3B, 4A and 4B, this hexagonal shape facilitates gripping by the walls 36 of chamber 22. Flared proximal end 16 is constructed in this hexagonal configuration by telescopically inserting proximal end 16 of catheter tubing 60 on a conical appendage 66 contained in a hexagonal aperture 68 as seen in FIGURE 4. The square edges of the hexagonal configuration makes movement of catheter 14 more difficult in chamber 22. Hexagonal aperture 68 in heated die 106 is provided for flared end 16. As seen in FIGURE 4A, catheter 14 is inserted into hexagonal opening 68, which is heated, having conical appendage 66 contained therein. This causes flared end 16 to melt and conform to the configuration of hexagaonal opening 68. After removal, as seen in FIGURES 3A and 4B, flared end 16 takes a hexagonal configuration conforming to hexagonal opening 68.

As an additional means of encapsulating flared proximal end 16, the flared proximal end 16 is also formed in a C-configuration 70 in cross section as best seen in FIGURE 3B. This C-shaped configuration 70 acts as a hook to prevent axial movement of catheter 14 during use. The C-shaped configuration 70 also produces a thickened wall portion 72 proximate flared proximal end 16. This further compresses flared proximal end 16 against the interior of walls 36 and chamber 22 in the area 40 behind and around flared proximal end 16. Again, tight gripping of catheter 14 without occluding lumen 20 is thereby produced.

An alternative method of flaring may be seen in FIGURE 3C. A pair of longitudinal heated dies 74 and 76 have a gap 78 between them. Flared proximal end 16 is placed in gap 78 between heated dies 74 and 76. The heat causes the flared proximal end 16 to curl, as shown in the drawing. Proper time and temperature control the degree of curl.

As best seen in FIGURES 5 - 8 of the drawings, The present invention also comprises a method of manufacturing a catheter 14 having a catheter hub 12 insert molded about the proximal end thereof. As illustrated in FIGURE 5, catheter 14 is then mounted on tapered core pin 80 which has an upward tubular section 82 of substantially the same outside diameter 84 as the inside diameter of lumen 18. Core pin 80 also has a lower tapered section 86 of increasing outside diameter. This lower tapered section 86 is used to form the lumen 20 in the proximal end 42 of catheter hub 12.

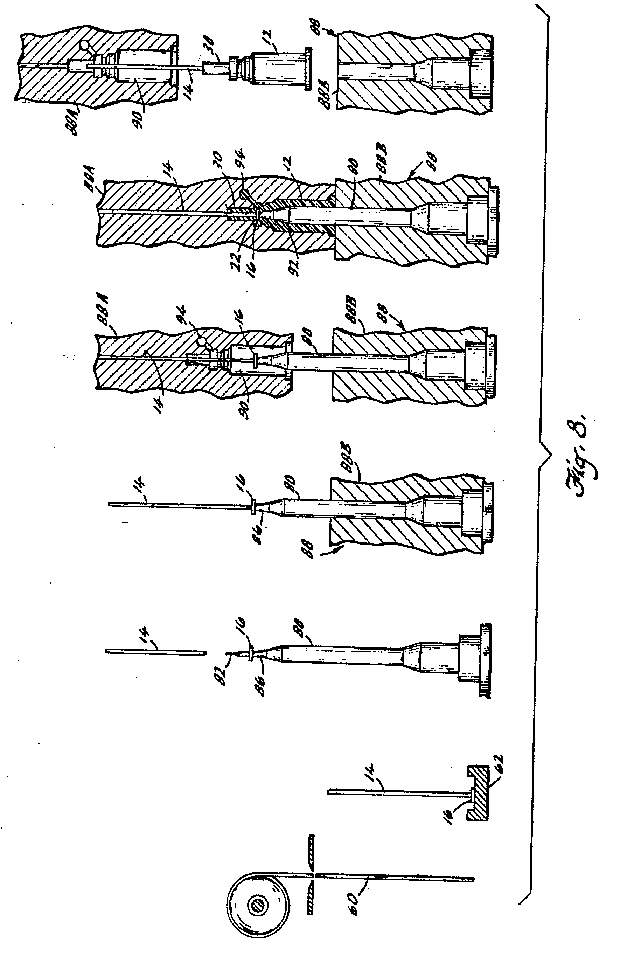

Referring to FIGURES 6 - 8, catheter 14 and core pin 80 are inserted into an injection mold 88 having a plurality of cavities 90 shaped as the exterior of catheter hub 12. Annular projection 26 is also formed in cavity 90 proximate the flared proximal end of catheter 14. Mold 88 consisting of mold halves 88A and 88B is then closed with catheter 14 in the desired position. (It should be noted in this regard that while mold 88 is shown as opening horizontally, a vertically

-13-

opening mold, i.e., opening along center line A-A of
FIGURE 1, may also be utilized.) A quantity of heated
plastic material 92 is injected into cavity 90 and about
catheter 12. The plastic material 92 is then allowed to
cool so as to form a catheter hub 12 about catheter 14
with chamber 22 formed about and encapsulating flared
proximal end 16 so as to mechanically grip the catheter
14 in the manner previously described. Mold 88 is then
opened and core pin 80, catheter hub 12 and catheter 14
are removed from mold 88. Catheter hub 12 with catheter
14 affixed therein is then removed from or stripped from
core pin 80.

An additional feature of the present method is
the positioning of the gate 94 of injection mold 88 at a
45° angle from chamber 22. This angle forces catheter
14 upward on core pin 80 into its proper position 96,
against tapered section 86 when heated plastic 92 is
injected. This also causes the flared proximal end 16
of catheter 14 to be encapsulated with heated plastic
material 92 and to compress the heated plastic about
flared proximal end 16 prior to the filling of the
remainder of cavity 90. This constitutes the first
stage of filling cavity 90. Following such filling the
catheter hub is complete. The means by which this
two-stage filling of cavity 90 is accomplished is that
the inside diameter 98 of cavity 90 and proximate and
immediately behind chamber 22 is decreased so as to form
the previously mentioned pinched waist portion 56. This
pinched waist constricts the flow of plastic material to
the rear of the mold 88. As a result, heated plastic 92
is compressed about flared proximal end 16, thereby
causing catheter hub 12 to tightly grip flared proximal
end 16.

FIGURE 8 of the drawings shows a schematic
diagram of the method of manufacturing the present

-14-

improved catheter assembly 10. In the first portion of FIGURE 8, catheter tubing 60 is cut to the desired length. In the second portion, catheter 14, which is thereby created has proximal end 16 pressed against heated die 62 so as to form a flared configuration 16. In the third sequence, the flared proximal end 16 of catheter 14 is placed on the tapered core pin 80, specifically on upper tubular section 82, until flared end 16 comes into abutment with lower tapered section 86. In the fourth segment, core pin 80 is inserted into mold 88. In the fifth segment, mold 88 is closed. In the sixth segment, heated plastic material 92 is inserted through gate 94 into cavity 90. Catheter hub 12 is thereby formed, with flared proximal end 16 being encapsulated within chamber 22 and tubular projection 30 tightly gripping the area adjacent flared proximal end 16. Following cooling of heated plastic material 92, as seen in FIGURES 7 and 8, mold 88 is opened and core pin 90 is removed. Catheter hub 12 and catheter 14 are then stripped from core pin 80.

The foregoing description and drawings merely explain and illustrate the invention and the invention is not so limited thereto, except insofar as those who are skilled in the art and have the disclosure before them are able to make modifications and variations therein without departing from the scope of the invention.

-15-

CLAIMS

1. An improved catheter assembly including a catheter hub and catheter comprising:

a flexible catheter having a flared proximal end, and an axial bore extending therethrough; and

a catheter hub having a lumen extending axially therethrough, said flexible catheter being fixedly positioned within said lumen, said catheter hub defining a chamber integrally formed therein, said flared proximal end of said catheter being tightly gripped and sealed by means of said catheter hub about the exterior of said flared proximal end, but allowing the flow of fluid through said lumen and said axial bore.

2. The catheter assembly as disclosed in Claim 1 and further comprising:

an annular projection integrally formed and circumferentially disposed about said catheter hub, said annular projection defining said chamber therein, wherein the wall thickness of said catheter hub about said flared proximal end and thereby the encapsulation of said flared proximal end within said chamber may be precisely controlled.

3. The catheter assembly as disclosed in Claim 1 and further comprising:

a substantially tubular projection integrally formed and extending from the distal end of said catheter hub, said tubular projection being of a reduced wall thickness relative to the remainder of said catheter hub so as to reduce shrinkage during manufacture, thereby improving gripping of said catheter by said catheter hub.

4. The catheter hub and catheter of Claim 1 wherein the exterior of said flared proximal end is encapsulated by said walls of said chamber, but said axial bore of said catheter is unobstructed and open to said lumen of said catheter hub whereby flow of fluid through said lumen is facilitated and seepage of fluid around said flared proximal end is prevented.

5. The catheter hub and catheter as disclosed in Claim 1 wherein said lumen of said catheter hub decreases in inside diameter proximate said flared proximal end so that the area behind and around said flared proximal end is filled with the material of the wall of said chamber, thereby encapsulating said flared proximal end.

6. The insert molded catheter hub and catheter of Claim 1 wherein said lumen conically tapers proximate the proximal end of said catheter hub so as to facilitate telescopic insertion of and connection to a tapered needle hub having a needle affixed therein, said needle thereby being positioned through said catheter hub and said catheter with the point of said needle extending sufficiently from said catheter to align with the bevel of the distal end of said catheter thereby forming a smooth transition between said distal end of said catheter and said point of said needle.

7. The catheter hub as disclosed in Claim 1 and further comprising:

a plurality of radial serrations axially disposed about the periphery of said catheter hub, constructed and arranged for gripping of said catheter hub during use.

-17-

8.  The catheter hub as disclosed in Claim 1 wherein said catheter is from 18 to 26 gauge in thickness and said catheter hub ranges from .025 - .035" plus or minus .005 depending on gauge in wall thickness proximate said chamber so as to reduce shrinkage of said catheter hub away from said catheter following molding.

9.  The catheter as disclosed in Claim 1 wherein said catheter is from 14-16 gauge in thickness and said catheter hub ranges from .025 - .035" plus or minus .005 depending on gauge in wall thickness proximate said chamber so as to reduce shrinkage of said catheter hub away from said catheter following molding.

10.  The catheter hub as disclosed in Claim 8 wherein the wall thickness of said catheter hub minimizes shrinkage of said walls of said catheter hub away from said catheter following molding thereby improving gripping of said catheter.

11.  The catheter hub as disclosed in Claim 9 wherein the wall thickness of said catheter hub minimizes shrinkage of said walls of said catheter hub away from said catheter following molding thereby improving gripping of said catheter.

12.  The catheter hub as disclosed in Claim 1 wherein said catheter hub is injection molded of a medical grade thermoplastic material.

13.  The catheter hub as disclosed in Claim 1 wherein said catheter is a medical grade tube of polyethylene terephthalate.

-18-

14. An improved catheter hub and catheter comprising:

a flexible catheter having a flared proximal end and an axial bore extending therethrough; and

a catheter hub having a lumen extending therethrough, said flexible catheter being fixedly positioned within said lumen, said catheter hub defining a chamber about said flared proximal end of said catheter being tightly gripped and sealed by means of said catheter hub about the periphery of said flared proximal end, but allowing the flow of fluid through said lumen and said axial bore.

15. The catheter hub as disclosed in Claim 13 wherein said catheter hub is bifurcated and is joined about said catheter.

16. The catheter hub as disclosed in Claim 13 wherein said flared proximal end is heat sealed to said chamber.

17. The catheter hub as disclosed in Claim 1 wherein said catheter is adhesively secured to said catheter hub.

18. The catheter hub as disclosed in Claim 1 and further including:

a pinched waist portion integrally formed in said catheter hub proximate and immediately behind said chamber, said pinched waist portion causing the rear portion of said chamber to be reduced in inside diameter thereby abutting against said flared proximal end of said catheter so as to prevent backward movement of said catheter during venipuncture.

19. A method of manufacturing a catheter having a catheter hub insert molded about the proximal end thereof comprising:

cutting a section of catheter tubing to a desired length, thereby forming a catheter;

abutting the proximal end of said catheter against a heated die having a flat base with a conical appendage extending substantially perpendicularly therefrom so as to cause said proximal end of said catheter to flare outward and to curl over;

removing said catheter from contact with said heated die;

mounting said catheter, flared end first, on a tapered core pin, said core pin having an upper tubular section of substantially the same outside diameter as the inside diameter of said catheter and a lower tapered section of increasing outside diameter;

inserting said catheter and core pin into an injection mold having a cavity shaped as a catheter hub and an enlarged chamber formed in said cavity proximate the flared proximal end of said catheter;

closing said mold;

introducing a quantity of heated plastic material thrugh a gate and into said cavity, and about said catheter;

cooling said plastic material so as to form a catheter hub about said catheter which mechanically grips said catheter;

opening said mold;

removing said core pin, catheter hub and catheter from said mold; and

removing said catheter hub and catheter from said core pin.

20. The method as disclosed in Claim 18 and further comprising the steps of:

positioning the gate of said injection mold at a 45° angle to said chamber so as to force said catheter upward on core pin and into its proper position against said tapered section when heated plastic is injected;

encapsulating said flared proximal end of said catheter in said heated plastic material; and

filling the chamber of said cavity with heated plastic material to form said catheter hub.

21. the method as disclosed in Claim 18 or 19 and further comprising the steps of:

decreasing the inside diameter of said cavity proximate and immediately behind said chamber, thereby constricting the flow of heated plastic therethrough so as to compress said plastic about said flared proximal end, thereby causing said catheter hub to tightly grip said flared proximal end.

22. The method of manufacturing a catheter as disclosed in Claim 18 and further comprising:

constructing said heated conical appendage in a hexagonal shape; and

forming said flared periphery of said catheter into a hexagonal configuration by telescopic insertion of said first end of said catheter tubing over said conical appendage;

said hexagonal configuration facilitating gripping of said flared proximal end of said catheter hub.

23. The catheter as disclosed in Claim 1 wherein said flared proximal end is formed in a hexagonal configuration so as to facilitate gripping thereof by said catheter hub.

-21-

24. The catheter hub as disclosed in Claim 1 wherein said flared proximal end is formed in a C configuration in cross section, so as to be encapsulated in said chamber, thereby preventing axial movement of said catheter during use.

25. The catheter as disclosed in Claim 1 wherein said flared proximal end further includes a thickened wall portion proximate said flared proximal end, said thickened wall portion being constructed and arranged to facilitate compression against the interior of said chamber, thereby more tightly gripping said catheter.

Fig. 2.

Fig. 3.

Fig. 3A.

Fig. 3B.

Fig. 3C.

Fig. 1.

1/3

0109657

Fig. 4.

Fig. 4 A.

Fig. 4 B.

Fig. 5.

Fig. 6.

Fig. 7.

2/3

0109657

0109657

Fig. 8.

# EUROPEAN SEARCH REPORT

European Patent Office

Application number

EP 83 11 1462

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-2 127 927 (BAXTER LABORATORIES) <br> * Page 5, lines 9-27; figure 3 * | 1-6,12 ,14 | A 61 M 25/00 <br> B 29 F 1/10C |
| Y | | 15,23 | |
| X | * Page 4, lines 32-37 * | 24 | |
| A | * Page 1, lines 12-16 * | 13 | |
| | --- | | |
| X | DE-A-1 491 725 (GIES) <br><br> * Whole document * | 1,3-7, 14,16, 17 | |
| | --- | | TECHNICAL FIELDS SEARCHED (Int. Cl. ³) |
| X,D | EP-A-0 001 106 (JOHNSON & JOHNSON) <br> * Page 8, lines 10-23; claim 6; figures 2,3 * | 1,3 | A 61 M <br> B 29 F |
| | --- | | |
| Y,D | US-A-3 802 433 (RAVEN) <br> * Figure 2 * | 15 | |
| | --- | | |
| Y | DE-A-1 491 743 (HENKE) <br> * Figures 2,3,4 * | 23 | |
| | --- | | |
| A,D | US-A-3 094 122 (GAUTHIER) | | |
| | ---    -/- | | |

The present search report has been drawn up for all claims

| Place of search <br> THE HAGUE | Date of completion of the search <br> 20-01-1984 | Examiner <br> KNAUER F.E. |
|---|---|---|

0109657

European Patent
Office

EUROPEAN SEARCH REPORT

Application number

EP 83 11 1462

| DOCUMENTS CONSIDERED TO BE RELEVANT | | | Page 2 | |
|---|---|---|---|---|
| Category | Citation of document with indication, where appropriate, of relevant passages | Relevant to claim | CLASSIFICATION OF THE APPLICATION (Int. Cl. ³) | |
| A | GB-A- 831 819 (EVERETT) | | | |
| A | DE-B-1 073 193 (BÜNDER GLAS GmbH) | | | |

The present search report has been drawn up for all claims

| Place of search | Date of completion of the search | Examiner |
|---|---|---|
| THE HAGUE | 20-01-1984 | KNAUER F.E. |

TECHNICAL FIELDS
SEARCHED (Int. Cl. ³)